(19) Europäisches Patentamt
European Patent Office
Office européen des brevets

(11) Veröffentlichungsnummer: **0 009 792**
**B1**

(12) # EUROPÄISCHE PATENTSCHRIFT

(45) Veröffentlichungstag der Patentschrift: **22.09.82**

(51) Int. Cl.³: **C 07 C121/75**, C 07 C120/00, A 01 N 53/00

(21) Anmeldenummer: **79103724.5**

(22) Anmeldetag: **01.10.79**
**Die Akte enthält technische Angaben, die nach dem Eingang der Anmeldung eingereicht wurden und die nicht in dieser Patentschrift enthalten sind.**

(54) **3-Chlorostyryl-2,2-dimethyl-cyclopropancarbonsäure-(4-fluoro-3-phenoxy-alpha-cyano-benzyl)-ester, Verfahren zu ihrer Herstellung und ihre Verwendung als Ektoparasitizide.**

(30) Priorität: **11.10.78 DE 2844271**

(43) Veröffentlichungstag der Anmeldung: **16.04.80 (Patentblatt 80/08)**

(45) Bekanntmachung des Hinweises auf die Patenterteilung: **22.09.82 Patentblatt 82/38**

(84) Benannte Vertragsstaaten: **BE CH DE FR GB IT NL**

(56) Entgegenhaltungen:
**EP - A - 0 000 345**
**DE - A - 2 738 150**

(73) Patentinhaber: **BAYER AG**
**Zentralbereich Patente, Marken und Lizenzen**
**D-5090 Leverkusen 1, Bayerwerk (DE)**

(72) Erfinder: **Fuchs, Rainer, Dr.**
**Roeberstrasse 8**
**D-5600 Wuppertal 1 (DE)**
Erfinder: **Stendel, Wilhelm, Dr.**
**In den Birken 55**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# 3-Chlorostyryl-2,2-dimethyl-cyclopropancarbonsäure-(4-fluoro-3-phenoxy-α-cyano-benzyl)-ester, Verfahren zu ihrer Herstellung und ihre Verwendung als Ektoparasitizide

Die Erfindung betrifft neue 3-Chlorostyryl-2,2-dimethyl-cyclopropancarbonsäure-(4-fluoro-3-phenoxy-α-cyano-benzyl)-ester, ein Verfahren zu ihrer Herstellung und ihre Verwendung als Mittel zur Bekämpfung von Ektoparasiten.

Es ist bereits bekannt, daß bestimmte Cyclopropancarbonsäure-phenoxybenzylester, wie z.B. 3-(2,2-Dichloro-vinyl)-2,2-dimethyl-cyclopropancarbonsäure-(3-phenoxy-α-cyano-benzyl)-ester und 3-(β-Chlor-β-phenylvinyl)-2,2-dimethyl-cyclopropancarbonsäure-α-cyano-3-phenoxybenzylester, zur Bekämpfung von Ektoparasiten verwendet werden können (vgl. DE-OS 2 601 743 und DE-OS 2 738 150).

Die Wirkung dieser Verbindungen ist jedoch, insbesondere bei niedrigen Wirkstoffkonzentrationen und Aufwandmengen, nicht immer zufriedenstellend.

Durch vorliegende Erfindung werden nun neue 3-Chlorostyryl-2,2-dimethyl-cyclopropancarbonsäure-(4-fluoro-3-phenoxy-α-cyano-benzyl)-ester der Formel I

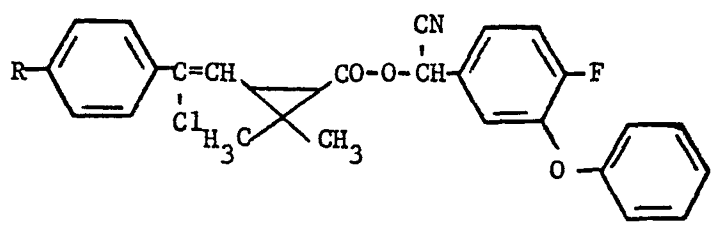

(I)

bereitgestellt, in welcher R für Wasserstoff oder Fluor steht.

Man erhält die neuen Verbindungen der Formel (I), wenn man 3-Chlorostyryl-2,2-dimethyl-cyclopropan-carbonsäurechloride der Formel II

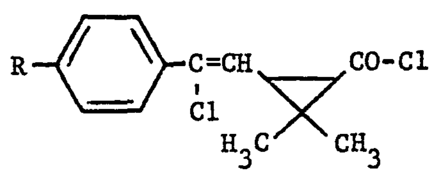

(II)

in welcher R die oben angegebene Bedeutung hat,
mit 4-Fluoro-3-phenoxy-benzaldehyd

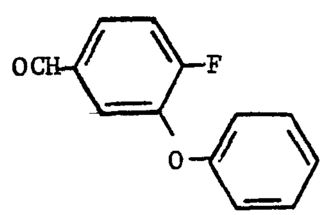

(III)

und wenigstens der äquimolaren Menge eines Alkalicyanids (vorzugsweise Natriumcyanid oder Kaliumcyanid), gegebenenfalls in Gegenwart eines Katalysators und gegebenenfalls unter Verwendung von Verdünnungsmitteln, umsetzt.

Die neuen 3-Chlorstyryl-2,2-dimethyl-cyclopropan-carbonsäure-(4-fluoro-3-phenoxy-α-cyano-benzyl)-ester (I) zeichnen sich durch hohe Wirksamkeit gegen Ektoparasiten aus.

Überraschenderweise zeigen die erfindungsgemäßen 3-Chloro-styryl-2,2-dimethyl-cyclopropancarbonsäure-(4-fluoro-3-phenoxy-benzyl)-ester eine erheblich höhere ektoparasitizide Wirkung als aus dem Stand der Technik bekannte Verbindungen ähnlicher Konstitution und gleicher Wirkungsrichtung.

Verwendet man beispielsweise 3-(2-Chloro-2-phenyl-vinyl)-2,2-dimethyl-cyclopropancarbonsäurechlorid, 4-Fluoro-3-phenoxy-benzaldehyd und Natriumcyanid als Ausgangsstoffe, so kann die Reaktion dieser Verbindungen durch folgendes Formelschema skizziert werden

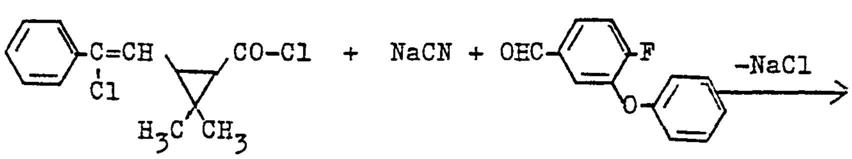

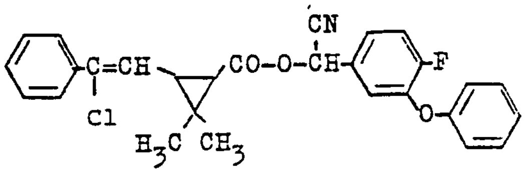

Die als Ausgangsverbindungen zu verwendenden 3-chlorostyryl-2,2-dimethyl-cyclopropancarbonsäurechloride sind bekannt oder können analog bekannten Verfahren hergestellt werden (vgl. DE-OS 2 738 150).

Als Beispiele seien genannt :

3-(2-Chloro-2-phenyl-vinyl)-2,2-dimethyl-cyclopropancarbonsäurechlorid und 3-(2-Chloro-2-(4-fluoro-phenyl)-vinyl)-2,2-dimethyl-cyclopropancarbonsäurechlorid.

Ebenso ist der als weitere Ausgangskomponente einzusetzende 4-Fluoro-3-phenoxy-benzaldehyd bekannt (vgl. DE-OS 2 709 264).

Das Verfahren zur Herstellung der erfindungsgemäßen neuen Verbindungen der Formel (I) wird bevorzugt unter Verwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl- Methylisopropyl- und Methylsiobutylketon sowie Nitrile wie Acetonitril und Propionitril.

Vorzugsweise werden von den genannten Verdünnungsmitteln die mit Wasser nicht mischbaren in Kombination mit Wasser als zweiter Lösungsmittelskomponente verwendet.

Als Katalysatoren verwendet man dann im allgemeinen Verbindungen, welche gewöhnlich bei Reaktionen in mehrphasigen Medien als Hilfsmittel zum Phasentransfer von Reaktanden dienen. Insbesonde seien Tetraalkyl- und Trialkyl-aryl-ammoniumsalze, wie z.B. Tetrabutylammoniumbromid und Trimethyl-benzyl-ammoniumchlorid, genannt.

Die Reaktionstemperatur kann innerhalb eines größeren Bereichs variiert werden. Im allgemeinen arbeitet man zwischen 0 und 100 °C, vorzugsweise bei 10 bis 50 °C. Das erfindungsgemäße Verfahren wird im allgemeinen bei Normaldruck durchgeführt, man kann aber auch unter erhöhtem Druck arbeiten.

Zur Durchführung des erfindungsgemäßen Verfahrens werden die Ausgangstoffe gewöhnlich in äquimolaren Mengen eingesetzt. Ein Überschuß der einen oder anderen Reaktionskomponente kann jedoch auch eingesetzt werden. Die Umsetzung wird im allgemeinen in einem oder mehreren Verdünnungsmitteln in Gegenwart eines Katalysators durchgeführt und das Reaktionsgemisch wird mehrere Stunden bei der erforderlichen Temperatur gerührt. Anschließend wird das Reaktionsgemisch mit Toluol/Wasser geschüttelt, die organische Phase abgetrennt, mit Wasser gewaschen und getrocknet. Nach Abdestillieren des Lösungsmittels erhält man dieneuen Verbindungen als Öle, welche nicht unzersetzt destilliert werden können. Sie können jedoch durch sogenanntes « Andestillieren », d.h. durch längeres Erhitzen unter vermindertem Druck auf mäßig erhöhtes Temperaturen von den letzten flüchtigen Anteilen befreit und auf diese Weise gereinigt werden. Zu ihrer Charakterisierung dienen die $^1H$-NMR-Spektren.

Die neuen Wirkstoffe der allgemeinen Formel (I) sowie ihre Salze weisen starke ektoparasitizide Wirkung auf, besonders gegen Acariden, die als tierische Ektoparasiten domestizierte Tiere, wie Rinder, Schafe und Kaninchen, befallen. Gleichzeitif haben die Wirkstoffe der allgemeinen Formel (I) nur eine geringe Warmblütertoxizität. Sie eignen sich daher gut zur Bekämpfung von tierischen Ektoparasiten aus der Klasse der Acariden. Darüber hinaus besitzen sie jedoch auch Wirkung gegen andere Acariden sowie gegen Insekten.

Beispielsweise seien genannt : Räudemilben, Läuse und Dipteren sowie deren Larven.

Als wirtschaftlich wichtige Ektoparasiten, die besonders in tropischen und subtropischen Ländern eine große Rolle spielen, seien genannt : die australische und südamerikanische Rinderzecke Boophilus microplus, die südafrikanische Rinderzecke Boophilus decoloratus, beide aus der Familie der Ixodidae, sowie die Rinder- und Schafzecken.

Im Laufe der Zeit sind insbesondere Zecken gegen die als Bekämpfungsmittel bisher verwendeten Phosphorsäureester und Carbamate resistent geworden, so daß der Bekämpfungserfolg in vielen Gebieten in wachsendem Maße infrage gestellt wird. Zur Sicherung einer wirtschaftlichen Viehhaltung in den Befallsgebieten besteht ein dringender Bedarf an Mitteln, mit denen alle Entwicklungsstadien, also Larven, Nymphen, Metanymphen und Adulti auch resistenter Stämme, beispielsweise des Genus Boophilus sicher bekämpft werden können. In hohem Maße gegen die bisher verwendeten Phosphorsäureester resistent sind beispielsweise in Australien der Mackay-Stamm, der Biarra-Stamm und der Mt-Alford-Stamm von Boophilus microplus.

Die erfindungsgemäßen Wirkstoffe gemäß Formel (I) sind sowohl gegen die normalempfindlichen, als auch gegen die resistenten Stämme, z.B. von Boophilus, gleich gut wirksam. Sie wirken in üblicher Applikation am Wirtstier sowohl direkt auf alle am Tier parastierenden Formen als auch stark ovizid auf die adulten Formen, sodaß der Vermehrungscyclus der Zecken sowohl in der parasitischen Phase auf

dem Tier als auch in der nicht parasitären Phase unterbrochen wird. Die Eiablage wird unterbunden, die Entwicklung und das Schlüpfen inhibiert.

Je nach der vorgesehenen Applikationsform können die neuen Wirkstoffe in die praxisüblichen Formulierungen übergeführt werden, wie beispielsweise Lösungen, Emulsionen, Suspensionen, Pulver, Pasten und Granulate. Diese werden in bekannter Weise hergestellt, z.B. durch Vermischen der Wirkstoffe mit Streckmitteln, d.h. flüssigen Lösungsmitteln und/oder Trägerstoffen, gegebenenfalls unter Mitverwendung von oberflächenaktiven Mitteln, also Emulgier- und/oder Dispergiermitteln, wobei z.B. im Falle der Verwendung von Wasser als Streckmittel gegebenenfalls organische Lösungsmittel als Hilfslösungsmittel verwendet werden können.

Als Lösungsmittel kommen z.B. infrage : Aromaten (z.B. Xylol, Benzol, Orthodichlorbenzol, Trichlorbenzol) Paraffine (z.B. Erdölfraktionen), Alkohole (z.B. Methanol, Äthanol, Isopropanol, Butanol), stark polare Lösungsmittel wie Dimethylformamid, N-Methyl-pyrrolidon, Dimethylsulfoxid sowie auch Wasser.

Als feste Trägerstoffe seien genannt : natürliche Gesteinsmehle (z.B. Kaoline, Tonerden, Talkum, Kreide) synthetische anorganische Trägerstoffe (z.B. hochdisperse Kieselsäure, Silikate) ; als Emulgiermittel : sowohl nichtionogene als auch anionische oder kationische Emulgatoren wie z.B. Polyoxyäthylen-Fettsäureester, Polyoxyäthylen-Fettalkoholäther, z.B. Alkyl-aryl-polyglykoläther ; Alkylsulfonate und Arylsulfonate, quatäre Ammoniumsalze mit längeren Alkylresten. Als Dispergiermittel seien genannt : Lignin, Sulfitablaugen und Methylcellulose.

Die Formulierungen enthalten im allgemeinen zwischen 0,1 und 95 Gew.-% Wirkstoff, vorzugsweise zwischen 0,5 bis 90 Gew.-%. Die Anwendungskonzentrationen werden aus den Formulierungen (s.o.) durch Verdünnen mit Wasser hergestellt. Sie können, je nach der Anwendungsform, in einem großen Bereich variiert werden und liegen zwischen 10 und 50 000 ppm (g/g), vorzugsweise zwischen 50 und 500 ppm.

Die Applikation erfolgt in üblicher Weise, z.B. durch Besprühen (spray), Gießen (pour on), Vernebeln oder durch Bad (dip).

Den Formulierungen oder den anwendungsfertigen Lösungen können noch sonstige Hilfsmittel oder Wirkstoffe, wie Desinfektionsmittel oder speziell geeignete Insektizide, zugemischt werden.

Die wäßrigen Lösungen bzw. Emulsionen der erfindungsgemäßen Wirkstoffe besitzen unter Praxisbedingungen eine gute Stabilität, sodaß die gebrauchsfertigen Anwendungsformen auch bei längerem Stehen und in einem pH-Bereich von 7-9 drei Monate und länger wirksam bleiben.

## Herstellungsbeispiele

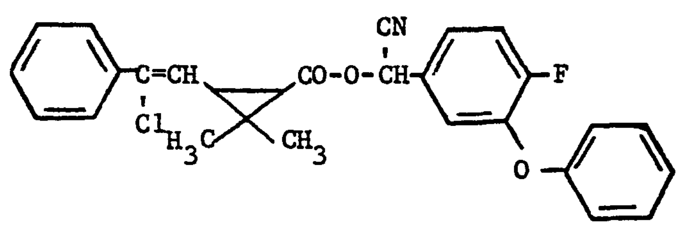

A

8,1 g (0,03 Mol) 3-(2-Chloro-2-phenyl-vinyl)-2,2-dimethylcyclopropan-carbonsäurechlorid und 6,5 g (0,03 Mol) 4-Fluoro-3-phenoxy-benzaldehyd werden tropfenweise unter Rühren bei 20 bis 25 °C zu einer Mischung aus 2,25 g Natriumcyanid, 3,5 ml Wasser, 150 ml n-Hexan und 0,75 g Tetrabutylammoniumbromid gegeben. Das Reaktionsgemisch wird dann 4 Stunden bei 20 bis 25 °C gerührt, anschließend mit 300 ml Toluol verdünnt und zweimal mit je 300 ml Wasser gewaschen. Die organische Phase wird über Magnesiumsulfat getrocknet und das Lösungsmittel wird in Wasserstrahlvakuum abdestilliert. Letzte Lösungsmittelreste werden durch kurzes Andestillieren bei 60 °C/1 mbar entfernt. Man erhält 10,8 g (76 % der Theorie) 3-(2-Chloro-2-phenyl-vinyl)-2,2-dimethyl-cyclopropancarbonsäure-(4-fluoro-3-phenoxy-benzyl)-ester als zähes Öl.

Analog erhält man aus 3-(2-Chloro-2-(4'-fluorophenyl)-vinyl)-2,2-dimethyl-cyclopropan-carbonsäuredichlorid und 4-Fluoro-3-phenoxy-benzaldehyd die Verbindung

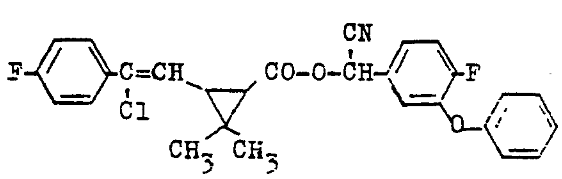

B

3-(2-Chloro-2-(4'-fluorophenyl)-vinyl)-2,2-dimethyl-cyclopropancarbonsäure-(4-fluoro-3-phenoxy)-benzylester als zähes Öl.

Die Struktur der beiden obengenannten Produkte ist durch die $^1$H-NMR-Spektren eindeutig erwiesen.

Characteristische $^1$H-NMR-Daten ($CDCl_3$/TMS) :

Verbindung (A) :

Aromatische-H : 2,30-3,15 τ (m/13H), benzyl-H : 3,55-3,76 τ (m/1H), vinyl-H : 4,0-4,38 τ (m/1H), cyclopropan-H : 7,19-8,50 τ (m/2H) und dimethyl-H : 8,50-8,9 τ (m/6H).

Verbindung (B) :

Aromatische-H : 2,28-3,21 τ (m/12H), benzyl-H : 3,50-3,78 τ (m/1H), vinyl-H : 4,1-4,43 τ (m/1H), cyclopropan-H : 7,26-8,50 τ (m/2H) und dimethyl-H : 8,50-9,0 τ (m/6H).

**Ansprüche**

1. 3-Chlorostyryl-2,2-dimethyl-cyclopropancarbonsäureester der Formel

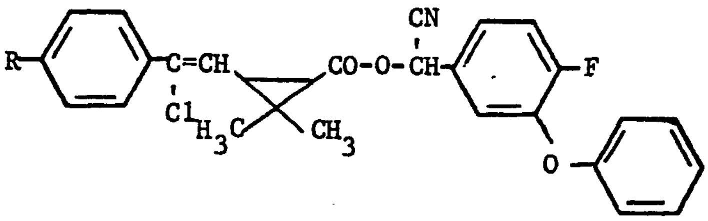

(I)

in welcher R für Wasserstoff oder Fluor steht.

2. Verfahren zur Herstellung von 3-Chlorostyryl-2,2-dimethyl-cyclopropancarbonsäureestern gemäß Anspruch 1, dadurch gekennzeichnet, daß man 3-Chlorostyryl-2,2-dimethyl-cyclopropan-carbonsäurechloride der Formel II

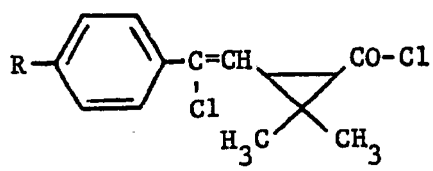

(II)

in welcher R die obengenannte Bedeutung hat,
mit 4-Fluoro-3-phenoxy-benzaldehyd

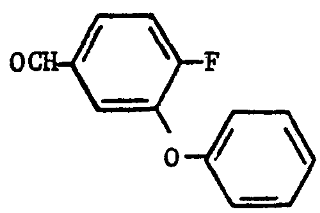

und wenigstens der äquimolaren Menge eines Alkalicyanids gegebenenfalls in Gegenwart eines Katalysators und gegebenenfalls unter Verwendung von Verdünnungsmitteln umsetzt und die entstehenden Reaktionsprodukte nach an sich bekannten Methoden isoliert.

3. Ektoparasitizides Mittel, gekennzeichnet durch einen Gehalt an mindestens einem 3-(Chlorostyryl-2,2-dimethyl-cyclopropancarbonsäureester gemäß Anspruch 1.

4. Verwendung von 3-Chlorostyryl-2,2-dimethylcyclopropancarbonsäureester gemäß Anspruch 1 bei der Bekämpfung von Ektoparasiten.

**Claims**

1. 3-Chlorostyryl-2,2-dimethyl-cyclopropanecarboxylic acid esters of the formula

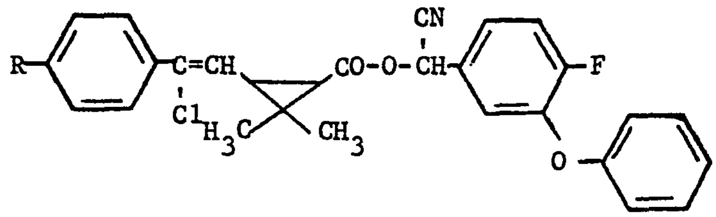

(I)

in which R represents hydrogen or fluorine.

2. Process for the preparation of 3-chlorostyryl-2,2-dimethyl-cyclopropanecarboxylic acid esters according to claim 1, characterised in that 3-chlorostyryl-2,2-dimethylcyclopropane-carboxylic acid chlorides of the formula II

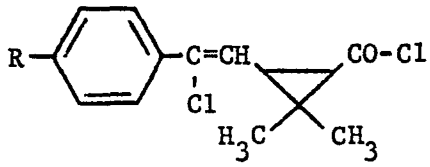

(II)

in which R has the meaning indicated above
are reacted with 4-fluoro-3-phenoxy-benzaldehyde

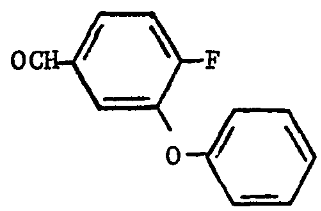

and at least an equimolar amount of an alkali metal cyanide, if appropriate in the presence of a catalyst and if appropriate using diluents, and the reaction products formed are isolated by methods which are in themselves known.

3. Ectoparasiticidal agent, characterised in that it contains at least one 3-chlorostyryl-2,2-dimethyl-cyclopropanecarboxylic acid ester according to claim 1.

4. Use of 3-Chlorostyryl-2,2-dimethylcyclopropanecarboxylic acid ester according to claim 1 in combating ectoparasites.

**Revendications**

1. Ester d'acide 3-chlorostyryl-2,2-diméthyl-cyclopropanecarboxylique de formule

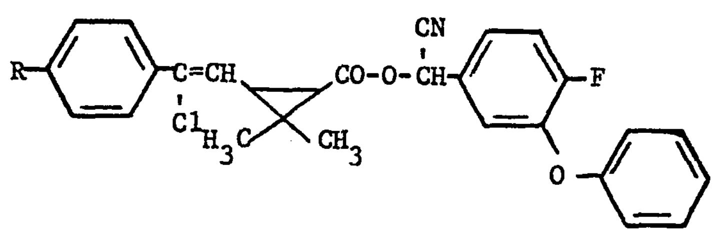

(I)

dans laquelle R représente un atome d'hydrogène ou de fluor.

2. Procédé de production d'esters de l'acide 3-chlorostyryl-2,2-diméthyl-cyclopropanecarboxyliques selon la revendication 1, caractérisé en ce qu'on fait réagir des chlorures d'acides 3-chlorostyryl-2,2-diméthyl-cyclopropane-carboxyliques de formule II

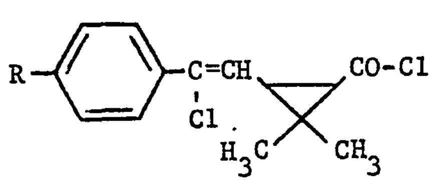

(II)

(dans laquelle R a le sens précité) avec le 4-fluoro-3-phénoxy-benzaldéhyde

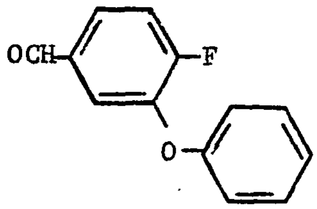

et avec au moins la quantité équimolaire d'un cyanure alcalin, éventuellement en présence d'un catalyseur et éventuellement en utilisant des diluants, et en ce qu'on isole, par des méthodes connues en elles-mêmes, les produits résultant de la réaction.

3. Ectoparasiticide, caractérisé en ce qu'il contient au moins un ester d'acide 3-chlorostyryl-2,2-diméthyl-cyclopropanecarboxylique selon la revendication 1.

4. Utilisation de l'ester de 3-chlorostyryl-2,2-diméthyl-cyclopropanecarboxylique selon la revendication 1 dans la lutte contre les ectoparasites.